# EUROPÄISCHE PATENTSCHRIFT

(11) **EP 1 807 529 B1**
(45) Veröffentlichungstag und Bekanntmachung des Hinweises auf die Patenterteilung: **19.08.2009**
(21) Anmeldenummer: 05805742.3
(22) Anmeldetag: 31.10.2005
(51) Int. Cl.: G01N 33/50, G01N 33/68, G01N 33/573

(54) **SCREENINGVERFAHREN ZUR FRÜHERKENNUNG DES ZEREBRALEN VASOSPASMUS**
SCREENING METHOD FOR THE EARLY DIAGNOSIS OF CEREBRAL VASOSPASM
PROCEDE DE DEPISTAGE PERMETTANT UN DEPISTAGE PRECOCE DU VASOSPASME CEREBRAL

(30) Priorität: 02.11.2004 DE 102004052889
(43) Veröffentlichungstag der Anmeldung: 18.07.2007
(73) Patentinhaber: Ruprecht-Karls-Universität Heidelberg, 69117 Heidelberg (DE)
(72) Erfinder: MAURER, Martin, 69121 Heidelberg (DE); KUSCHINSKY, Wolfgang, 69118 Heidelberg (DE); HAUX, Daniel, 69115 Heidelberg (DE); SAKOWITZ, Oliver, 68526 Ladenburg (DE); FELDMANN, Robert, 65193 Wiesbaden (DE); UNTERBERG, Andreas, 69118 Heidelberg (DE)
(74) Vertreter: Elbel, Michaela
(86) Internationale Anmeldenummer: PCT/EP2005/011804
(87) Internationale Veröffentlichungsnummer: WO 2006/048298

(56) Entgegenhaltungen:
- EP-A- 1 006 108
- MILES M F ET AL: "MECHANISMS OF NEURONAL ADAPTATION TO ETHANOL ETHANOL INDUCES HSC70 GENE TRANSCRIPTION IN NG108-15 NEUROBLASTOMAS X GLIOMA CELLS" JOURNAL OF BIOLOGICAL CHEMISTRY, Bd. 266, Nr. 4, 1991, Seiten 2409-2414, XP002362594 ISSN: 0021-9258
- DOHI K ET AL: "Transient elevation of serum bilirubin (a heme oxygenase-1 metabolite) level in hemorrhagic stroke: bilirubin is a marker of oxidant stress." ACTA NEUROCHIRURGICA. SUPPLEMENT. 2003, Bd. 86, 2003, Seiten 247-249, XP008058322 ISSN: 0065-1419
- MIWA KUNIHISA ET AL: "Increased oxidative stress with elevated serum thioredoxin level in patients with coronary spastic angina." CLINICAL CARDIOLOGY. APR 2003, Bd. 26, Nr. 4, April 2003 (2003-04), Seiten 177-181, XP008058263 ISSN: 0160-9289
- CIOLINO HENRY P ET AL: "Modification of proteins in endothelial cell death during oxidative stress" FREE RADICAL BIOLOGY AND MEDICINE, Bd. 22, Nr. 7, 1997, Seiten 1277-1282, XP002362596 ISSN: 0891-5849
- CHUANG DE-MAW ET AL: "Glyceraldehyde-3-phosphate dehydrogenase, apoptosis and neurodegenerative diseases" ANNUAL REVIEW OF PHARMACOLOGY AND TOXICOLOGY, [Online] Bd. 45, 7. September 2004 (2004-09-07), Seiten 269-290,C1, XP002362597 ISSN: 0362-1642
- DASTOOR Z ET AL: "Nuclear translocation and aggregate formation of heat shock cognate protein 70 (Hsc70) in oxidative stress and apoptosis." JOURNAL OF CELL SCIENCE. AUG 2000, Bd. 113 ( Pt 16), August 2000 (2000-08), Seiten 2845-2854, XP002362598 ISSN: 0021-9533 in der Anmeldung erwähnt
- MAZZOLA JENNIFER L ET AL: "Alteration of intracellular structure and function of glyceraldehyde-3-phosphate dehydrogenase: A common phenotype of neurodegenerative disorders?" NEUROTOXICOLOGY (LITTLE ROCK), Bd. 23, Nr. 4-5, Oktober 2002 (2002-10), Seiten 603-609, XP002363397 ISSN: 0161-813X
- DATABASE BIOSIS [Online] BIOSCIENCES INFORMATION SERVICE, PHILADELPHIA, PA, US; Dezember 1999 (1999-12), KIM JIN W ET AL: "Antisense oligodeoxynucleotide of glyceraldehyde-3-phosphate dehydrogenase gene inhibits cell proliferation and induces apoptosis in human cervical carcinoma cell lines" XP002363399 Database accession no. PREV200000149430

## Beschreibung

### Umfeld der Erfindung

Die vorliegende Erfindung betrifft das technische Feld von Screeningverfahren und Testsystemen. Insbesondere betrifft die Erfindung ein Verfahren zur in vitro Diagnose sowie ein Testsystem zur Erkennung eines erhöhten Vasospasmus-Risikos in einem Patienten. Daneben beschreibt die Erfindung die Verwendung von Glyzeraldehyd-3-phosphat-Dehydrogenase Protein (GAPDH) und Heatshock-cognate 70 kDa Protein (Hsc70) zur Diagnose eines erhöhten Vasospasmus-Risikos. Ferner ist ein Verfahren zum Identifizieren einer pharmazeutisch aktiven Substanz zur Prävention und/öder Behandlung von zerebralem Vasospasmus sowie ein Verfahren zum Screenen einer Substanzbibliothek zur Identifizierung einer pharmazeutisch aktiven Substanz zur Prävention und/oder Behandlung von zerebralem Vasospasmus umfasst.

### Hintergrund der Erfindung

Der Schlaganfall gehört zu den häufigsten Ursachen für Tod und Behinderung. Etwa 85 % der Schlaganfallpatienten erleiden Gefäßverschlüsse, sogenannte ischämische Schlaganfälle, während bei 15 % der Patienten eine Blutung im Gehirn die Ursache ihrer Erkrankung darstellt. Neben der Massenblutung in das Gehirnparenchym ist die Blutung in den Subarachnoidalraum, die sogenannte Subarachnoidalblutung (SAB), für die meisten Fälle verantwortlich. Die SAB trägt zu 6-8 % aller Schlaganfälle bei und ist für 22-25 % aller zerebrovaskulären Todesfälle verantwortlich (Dumont et al., Neurosurgery 53 (2003), 123-133; Diskussion 33-35).

Ursachen für die SAB sind zu 60 % intrakranielle Aneurysmen, zu 10 % arteriovenöse Gefäßfehlbildungen und zu 10 % seltenere Ursachen wie z. B. hämorrhagische Diathesen, Antikoagulanzien-Therapie, Tumoren oder Vaskulitiden. Etwa 20 % der Fälle bleiben ätiologisch ungeklärt. Schätzungsweise 1-12 Millionen Menschen tragen ein zerebrales Aneurysma in den Vereinigten Staaten von Amerika mit einer jährlichen Prävalenz für eine SAB von 30.000 Personen (Dumont et al., Neurosurgery 53 (2003), 123-133).

Pro Jahr erkranken etwa 7-20 von 100.000 Einwohnern an einer SAB (Daten für Nordamerika und Europa) (Dumont et al., Neurosurgery 53 (2003), 123-133). Die SAB ist mit einer hohen Mortalität behaftet, etwa 15 % der Patienten versterben noch vor Erreichen des Krankenhauses, weitere 15 % innerhalb der ersten 24 Stunden, weitere 15 % innerhalb der ersten 2 Wochen und nochmals 15 % innerhalb der nächsten 2 Monate. Nach 2 Jahren leben nur noch 15 % der SAB-Patienten, wobei die meist auch noch durch starke Behinderungen in ihrem täglichen Leben beeinträchtigt sind.

Neben den Einschränkungen des Lebens der Patienten sind die Kosten während der Krankenhausphase durch die lange Behandlung auf der Intensivstation sehr hoch. Hinzu kommt der volkswirtschaftliche Schaden durch Verdienstausfall und Berentung.

Die Hauptkomplikationen der SAB bestehen neben der Nachblutung und einem Okklusionshydrozephalus vor allem in einer sekundären Ischämie, die durch einen symptomatischen zerebralen Vasospasmus hervorgerufen wird. Synonym hierzu wird das verzögerte neurologische Defizitsyndrom genannt (delayed ischemic neurological deficit syndrome, DINDS) (Hijdra et al., Stroke 18 (1987), 1061-1067). Eine angiographisch nachgewiesene Gefäßverengung tritt als Komplikation in etwa 70 % der Fälle auf, ein klinisch manifester Vasospasmus in etwa 20-30 % der Fälle (Dumont et al., Neurosurgery 53 (2003), 123-133; Diskussion 33-35; Janjua und Mayer, Curr Opin Crit Care 9 (2003), 113-119). Frauen sind hiervon deutlich häufiger betroffen als Männer. Trotz Maximaltherapie entwickeln etwa 50 % der Vasospasmuspatienten einen zusätzlichen ischämischen Hirninfarkt. Das Maximum des symptomatischen Vasospasmus tritt etwa 7-14 Tage nach der SAB auf, so dass zwischen Blutungsereignis und Vasospasmus Zeit bleibt, therapeutisch-prophylaktisch einzugreifen.

Die Pathogenese des zerebralen Vasospasmus ist komplex. Es werden verschiedene Modelle und Einflussfaktoren diskutiert, unter anderem (1) freies Hämoglobin im Subarachnoidalraum; (2) Entzündungsreaktionen auf die Blutung vermittelt durch Zytokine wie TNF-α, IL-1α, IL-1β, IL-6 und IL-8, Immunglobulinen, NFkB, Leukozyten und Leukozytenadhäsionsmolekülen, COX-2, ET-1 und PARP; (3) ein gestörter NO-Stoffwechsel; (4) Veränderungen der Aktivität der Proteinkinase C; (5) die Bildung freier Radikale; (6) die Freisetzung von Prostaglandinen; (7) die Degradation von Filament-assoziierten Proteinen (Dietrich und Dacey, Neurosurgery 46 (2000), 517-530; Dumont et al., J Neurosurgery 96 (2002), 985-986; Diskussion 6-7; Dumont et al., Neurosurgery 53 (2003), 123-133; Janjua und Mayer, Curr Opin Crit Care 9 (2003), 113-119; Laher und Zhang, J Cereb Blood Flow Metab 21 (2001), 887-906; Sobey, Clin Exp Pharmacol Physiol 28 (2001), 926-929).

Die EP 1 122 317 A1 beschreibt einen monoklonalen Antikörper gegen GAPDH, ein Hybridom zu seiner Herstellung sowie die Verwendung dieses Antikörpers zur Behandlung verschiedener neurodegenerativer Erkrankungen wie beispielsweise Alzheimer Erkrankung, Parkinson Erkrankung und Creutzfeldt-Jacob Erkrankung.

Die WO 01/52890 A1 beschreibt einen Komplex aus einem Hitzeschockprotein und einem antigenen Molekül, das die Antigenität eines Antigens zeigt, das mit einer neurodegenerativen Störung assoziiert ist. Das Hitzeschockprotein kann das Hsp70 Protein sein. Als neurodegenerative Störungen sind unter anderem die Alzheimer Erkrankung, die Parkinson Erkrankung, die Creutzfeldt-Jacob Erkrankung, der Schlaganfall sowie Gehirntrauma genannt.

Zurzeit ist unbekannt, warum bei einigen Patienten nach einer SAB ein Vasospasmus auftritt und bei anderen nicht. Da das Maximum des Vasospasmus erst etwa 7-14 Tage nach SAB auftritt, bleibt davor Zeit; um einem Vasospasmus therapeutisch-prophylaktisch vorzubeugen. Voraussetzung dafür ist jedoch, die Patienten herauszufinden, die mit hoher Wahrscheinlichkeit einen Vasospasmus erleiden werden.

Ein Frühindikator für den Vasospasmus existiert nicht. Gegenwärtig wird ein Vasospasmus durch Veränderungen des klinischen Bildes, bildgebende Verfahren (CT, MRT), Blutflussuntersuchungen (Doppler-Sonografie) und durch Veränderungen metabolischer Parameter bei der zerebralen Mikrodialyse (Pyruvat, Laktat, Glutamat) (Berger et al., Nervenarzt 75 (2004), 113-123) erst nach seinem Auftreten erfasst.

Alle bisher genannten Verfahren erlauben die Diagnose erst nach Auftreten des Vasospasmus, so dass eine rechtzeitige therapeutische Intervention nicht möglich ist.

Somit besteht ein Bedarf an Verfahren zur in vitro Diagnose sowie Testsystemen zur Erkennung eines erhöhten Vasospasmus-Risikos. Es ist deshalb die Aufgabe der vorliegenden Erfindung ein Verfahren bereitzustellen, das die Früherkennung eines zerebralen Vasospasmus ermöglicht.

Die vorliegende Erfindung erlaubt, durch molekulare Indikatoren noch vor Auftreten des Vasospasmus eine Gefährdung der Patienten nach SAB zu erkennen, sie engmaschig zu überwachen und eine frühzeitige Prophylaxe einzuleiten.

### Zusammenfassung der Erfindung

Die vorliegende Erfindung betrifft ein in vitro Verfahren zur Diagnose eines erhöhten Vasospasmus-Risikos eines Patienten, umfassend die Schritte:
(a) Bereitstellen einer Probe des Patienten;
(b) Bestimmen der Menge an Glyzeraldehyd-3-phosphat-Dehydrogenase Protein (GAPDH) und Heat-shock-cognate 70 kDa Protein (Hsc70) in der Testprobe;
(c) Vergleichen der Menge an GAPDH und Hsc70 mit einem Referenzwert;
wobei eine gegenüber dem Referenzwert erhöhte GAPDH Menge und eine gegenüber dem Referenzwert verringerte Hsc70 Menge ein erhöhtes Vasospasmus-Risiko anzeigt.

Ferner betrifft die vorliegende Erfindung ein Testsystem zur Erkennung eines erhöhten Vasospasmus-Risikos nach einem Schlaganfall, umfassend:
(a) wenigstens jeweils einen Antikörper gegen GAPDH und Hsc70;
(b) wenigstens einen sekundären Antikörper zum Nachweis von jeweils Antikörper/GAPDH und/oder Antikörper/Hsc70 Komplexen;
(c) Mittel zur Quantifizierung der Komplexe;
wobei eine gegenüber einem Referenzwert erhöhte Menge an GAPDH und eine gegenüber einem Referenzwert erniedrigte Menge an Hsc70 ein erhöhtes Vasospasmus-Risiko anzeigt.

Darüber hinaus betrifft die Erfindung ein in vitro Verfahren zum Identifizieren einer pharmazeutisch aktiven Substanz zur Prävention und/oder Behandlung von zerebralem Vasospasmus, umfassend die Schritte:
(a) Bereitstellen einer Zelle, die GAPDH und Hsc70 exprimiert;
(b) In Kontakt bringen einer Testsubstanz mit der Zelle;
(c) Messen der Menge an GAPDH und Hsc70;
wobei eine Reduktion der Menge an GAPDH und eine Erhöhung der Menge an Hsc70 die Testsubstanz als pharmazeutisch aktive Substanz qualifiziert.

Ferner ist ein in vitro Verfahren zum Screenen einer Substanzbibliothek zur Identifizierung einer pharmazeutisch aktiven Substanz zur Prävention und/oder Behandlung von zerebralem Vasospasmus umfasst, welches die Schritte umfasst:
(a) Bereitstellen von Testproben, die Zellen enthalten, die GAPDH und Hsc70 exprimieren;
(b) Messen der Ausgangs-Expressionsmenge an GAPDH und Hsc70;
(c) In Kontakt bringen der Testproben mit Kandidaten der Substanzbibliothek;
(d) Identifizieren einer Testprobe, die verglichen mit der Ausgangs-Expressionsmenge weniger GAPDH und mehr Hsc70 exprimiert;
(e) Bestimmen des Kandidaten der Substanzbibliothek, der mit der Testprobe in Kontakt gebracht wurde,
wobei der Kandidat der Substanzbibliothek eine pharmazeutisch aktive Substanz ist.

### Kurzbeschreibung der Figuren

Fig. 1 zeigt zweidimensionale Elektropherogramme aus menschlichem Mikrodialysat für die asymptomatischen SAB-Patienten (linke Hälfte) und die Patienten mit Vasospasmus (rechte Hälfte). Differenziell exprimierte Proteingruppen wurden durch hierarchische Clusteranalyse identifiziert und sind markiert (Pfeile) für (A) Heat-shock-cognate-70 kDa-Protein (Hsc70) und (B) Glyzeraldehyd-3-phosphat-Dehydrogenase (GAPDH).
Fig. 2 zeigt eine hierarchische Clusteranalyse der Proteomprofile. Der Cluster-Algorithmus sortiert die Proteinpunkte gemäß ihrem Expressionsprofil. Die Experimentnummern finden sich oben (n=24), die Spotnummern rechts. Differenzielle Cluster sind markiert für Hsc70 und GAPDH.
Fig. 3 zeigt eine vermehrte Expression von GAPDH im Mikrodialysat vasospastischer Patienten nach SAB. Die durchschnittliche Proteinkonzentration der GAPDH war um Faktor 3,68 ± 1,09 in den Patienten der Vasospasmusgruppe gegenüber den asymptomatischen Kontrollpatienten vermehrt.
Fig. 4 zeigt eine verminderte Expression von Hsc70 im Mikrodialysat vasospastischer Patienten nach SAB. Die durchschnittliche Proteinkonzentration des Hsc70 war um Faktor 0,47 ± 0,18 in den Patienten der Vasospasmusgruppe gegenüber den asymptomatischen Kontrollpatienten vermindert.
Fig. 5 zeigt Veränderungen im Mikrodialysat vor Auftreten des Vasospasmus. Im Mittel wurden Proteinveränderungen im Mikrodialysat 2,4 ± 2,1 Tage vor dem klinischen Auftreten des Vasospasmus gefunden.

### Detaillierte Beschreibung der Erfindung

Die vorliegende Erfindung betrifft zunächst in vitro Verfahren zur Diagnose eines erhöhten Vasospasmus-Risikos eines Patienten, umfassend die Schritte:
(a) Bereitstellen einer Probe des Patienten;
(b) Bestimmen der Menge an Glyzeraldehyd-3-phosphat-Dehydrogenase Protein (GAPDH) und Heat-shock-cognate 70 kDa Protein (Hsc70) in der Testprobe;
(c) Vergleichen der Menge an GAPDH und Hsc70 mit einem Referenz wert;
wobei eine gegenüber dem Referenzwert erhöhte GAPDH Menge und eine gegenüber dem Referenzwert verringerte Hsc70 Menge ein erhöhtes Vasospasmus-Risiko anzeigt.

Die vorliegende Erfindung hat durch Proteomanalyse basierend auf zweidimensionaler Gelelektrophorese und Massenspektrometrie zwei neue Markerproteine für den zerebralen Vasospasmus im Mikrodialysat identifiziert. Dies sind die Glyzeraldehyd-3-phosphat-Dehydrogenase (GAPDH) und das Protein Heat-shock-cognate-70 kDa (Hsc70). Die Konzentration der GAPDH war dabei um den Faktor 3,68 ± 1,09 (Daten aus 4 Protein-Isoformen) auf mehr als das Dreifache in den Patienten der Vasospasmusgruppe gegenüber den asymptomatischen Kontrollpatienten erhöht, während die Konzentration des Hsc70 um den Faktor 0,47 ± 0,18 (Daten aus 9 Protein-Isoformen) auf etwa die Hälfte vermindert war.

Die beiden genannten Proteine wurden bisher nicht mit zerebralem Vasospasmus und speziell seiner Frühdiagnose in Verbindung gebracht.

Die wesentlichen Vorteile der vorliegenden Erfindung bestehen darin, dass noch vor Eintreten der klinischen Verschlechterung das Risiko, einen Vasospasmus nach SAB zu entwickeln, beurteilt werden und entsprechend frühzeitige Prophylaxe- und/oder Behandlungsschritte eingeleitet werden können.

Neben den bekannten enzymatischen Funktionen der GAPDH im Glukosestoffwechsel konzentrierte sich das Interesse an diesem Protein in den letzten Jahren verstärkt auf die Rolle bei Zelltod und Apoptose, Interaktion mit NO, Endozytose, Mikrotubulus-Bündelung, Phosphotransferase-Aktivität, Regulation der Genexpression, Transport von intranukleärer RNA, DNA-Replikation und -Reparatur sowie translationalen Regulationsvorgängen (Sirover, J Cell Biochem 66 (1997), 133-140; Sirover, Biochim Biophys Acta 1432 (1999), 159-184).

Verminderte GAPDH-Aktivität findet sich auch bei zahlreichen neurodegenerativen Erkrankungen wie z. B. Morbus Alzheimer, Morbus Huntington, der dentatorubralen pallidoluysischen Atrophie und anderen CAG-Triplett-Erkrankungen. GAPDH ist außerdem das Zielmolekül von Deprenyl, einer Substanz, die zur Theapie des Morbus Parkinson eingesetzt wird und die neuroprotektiv wirkt (Kragten et al., J Biol Chem 273 (1998), 5821-5828; Tatton et al., J Neural Transm 110, (2003), 509-515).

Durch die nukleäre Translokation kommt es vermutlich zu den apoptotischen Signalen, die für den Zelluntergang von Neuronen verantwortlich sind (Chuang und Ishitani, Nat Med 2 (1996), 609-610; Sawa et al., Proc Natl Acad Sci USA 94 (1997), 11669-11674; Sunaga et al., Neurosci Lett 200 (1995), 133-136; Tatton et al., J Neural Transm Suppl (2000), 77-100). Außerdem ist die Expression von GAPDH durch Hypoxie regulierbar (Graven et al., Am J Physiol 274 (1998), C347-55; Graven et al., J Biol Chem 269 (1994), 24446-24453). GAPDH bildet mit TOAD64, der Enolase-χ, der Aldolase C und Hsc70 Proteinkomplexe (Bulliard et al., Biochem J 324 (Pt 2) (1997), 555-563), die für Membranfusion und intrazelluläre Signalwege eine Rolle spielen. GAPDH ist außerdem durch Oxyhämoglobin stimulierbar (Brookes et al., FEBS Lett 416 (1997), 90-92).

Hsc70 gehört zur Gruppe der molekularen Chaperone der Hsp70-Familie. Diese Proteinfamilie findet sich in Zellkern und Zytosol und dient als Faltungshilfe für wachsende Polypeptidketten. Sie verhindern außerdem eine Akkumulation fehlgefalteter Proteine. Hsc70 findet sich sowohl in Neuronen als auch Gliazellen des Gehirns (Myung et al., Mol Genet Metab 80 (2003), 444-450) und bildet Komplexe mit dem Heat-shock-Protein 90 kDa (Hsp90), der Glyzeraldehyd-3-phosphat-Dehydrogenase (GAPDH), der Leucinaminopeptidase (LAP) und der Adenosylhomocysteinase (Nakamura et al., Biochem Biophys Res Commun 290 (2002), 858-864). Oxidativer Stress aufgrund hypoxisch-ischämischer Bedingungen führt zu nukleärer Translokation des Hsc70 (Dastoor und Dreyer, J Cell Sci 113 (Pt 16) (2000), 2845-2854) und der GAPDH (Sawa et al., Proc Natl Acad Sci USA 94 (1997), 11669-11674), die beide apoptotische Vorgänge in den betroffenen Zellen fördern.

Ohne durch irgendeine Theorie beschränkt zu sein, nehmen die Erfinder der vorliegenden Erfindung an, dass zwischen beiden Proteinen GAPDH und Hsc70, außer ihrer Fähigkeit aneinander zu binden, auch ein funktioneller Zusammenhang besteht. Dieser Zusammenhang könnte über den NO-Metabolismus gegeben sein. Beide Proteine reagieren mit NO, und Reaktionsprodukte des NO (NOₓ) sind im Mikrodialysat von Patienten mit Vasospasmus nach SAB vermindert im Vergleich zu nicht-vasospastischen Kontrollpatienten (Sakowitz et al., J Cereb Blood Flow Metab 21 (2001), 1067-1076).

In einer bevorzugten Ausführungsform der Erfindung ist die Probe des Patienten ein zerebrales Mikrodialysat, Liquor cerebrospinalis, Blutplasma oder Blutserum.

Das Mikrodialysat kann durch Mikrodialyse gewonnen werden. Die Mikrodialyse ist eine Verfahren, mit dem Bestandteile der Extrazellulärflüssigkeit in lebendem Gewebe gesammelt werden (Benveniste, J Neurochem 52 (1989), 1667-1679; Ungerstedt, Measurement of neurotransmitter release in vivo. 6, Marsden CA Hrsg. Chishester, Wiley (1984), 81-105; Ungerstedt, J Int Med 230 (1991), 365-373). Sie wurde bei Schlaganfallpatienten hauptsächlich zur Verlaufskontrolle von Neurotransmittern und kleinen Metaboliten des Gehirns eingesetzt (Berger et al., Nervenarzt 75 (2004), 113-123).

Mehrere Studien haben bisher die zerebrale Mikrodialyse eingesetzt, um den Verlauf nach SAB zu untersuchen (Peerdeman et al., J Neurol 250 (2003), 797-805). Als Marker eines gestörten zerebralen Metabolismus auf Grund des symptomatischen Vasospasmus zeigten sich insbesondere das Glutamat, Laktat und der Laktat-Pyruvat-Quotient (Persson und Hillered, J Neurosurg 76 (1992), 72-80; Saveland et al., Neurosurgery 38 (1996), 12-19; Sarrafzadeh et al., Crit Care Med 30 (2002), 1062-1070; Unterberg et al., J Neurosurg 94 (2001), 740-749; Sarrafzadeh et al., Stroke 35 (2004), 638-643).

Die zerebrale Mikrodialyse ist bislang jedoch auf spezialisierte Zentren mit den nötigen technischen Voraussetzungen beschränkt, wodurch die Patientenzahl begrenzt bleibt (Janjua und Mayer, Curr Opin Crit Care 9 (2003), 113-119). Außerdem ist Mikrodialyse als lokale Messungmethode bei ungenau platziertem Messkatheter oft nicht in der Lage, eine ischämische metabolische Störung auf G rund des Vasospasmus frühzeitig anzuzeigen (Sarrafzadeh et al., Curr Neurol Neurosci Rep 3 (2003), 517-523).

In einer weiteren bevorzugten Ausführungsform tritt das erhöhte Vasospasmus-Risiko nach einer Subarachnoidalblutung (SAB) oder nach einer Gehirnmassenblutung auf.

In einer weiteren Ausführungsform erfolgt die Bestimmung der Menge an GAPDH und Hsc70 durch eine Proteinkonzentrationsbestimmung.

Die Proteinkonzentrationsbestimmung kann vorteilhafterweise durch einen Antikörper oder durch zweidimensionale Gelelektrophorese erfolgen. Es können sowohl monoklonale als auch polyklonale Antikörper mit einer geeigneten Spezifität für GAPDH und Hsc70 verwendet werden. Monoklonale Antikörper sind aufgrund ihrer höheren Spezifität und einfachen Herstellung bevorzugt. Synthetisch erzeugte Antikörper können ebenfalls verwendet werden.

Die zweidimensionale Gelelektrophorese ist ein Verfahren zur simultanen Untersuchung sehr vieler Proteine in einer Probe (Görg et al., Electrophoresis 21 (2000), 1037-1053; Rabilloud, Proteomics 2 (2002), 3-10). Sie ist eine der wichtigsten Verfahren der Proteomforschung. Mit dem Proteom wird die Gesamtheit aller Proteine einer biologischen Probe, einer Zelle, eines Organs oder eines Organismus bezeichnet.

Besonders vorteilhaft ist in diesem Zusammenhang die Proteinbestimmung durch zweidimensionale Gelelektrophorese, wobei insbesondere ein Verfahren verwendet werden kann, mit dem durch eine Proteomuntersuchung die im Mikrodialysat enthaltenen Proteine dargestellt und untersucht werden können.

Dieses Verfahren beruht auf der zweidimensionalen Gelelektrophorese in Kombination mit der Massenspektrometrie, womit von den Erfindern der vorliegenden Erfindung eine Referenzdatenbank und eine Referenzproteinkarte für humanes zerebrales Mikrodialysat erstellen wurde (Maurer et al., Proteome Sci 1 (2003), 7).

In einer weiteren Ausführungsform der Erfindung ist der Vasospasmus ein zerebraler Vasospasmus.

Ein weiterer Gegenstand der vorliegenden Erfindung ist die in vitro Verwendung von GAPDH und Hsc70 zur Diagnose eines erhöhten Vasospasmus-Risikos. Der Vasospasmus ist bevorzugt ein zerebraler Vasospasmus.

GAPDH und/oder Hsc70 können bevorzugt zur Herstellung eines Arzneimittels oder eines Medizinproduktes zur Prävention und/oder Therapie von zerebralem Vasospasmus verwendet werden.

Ein weiterer Gegenstand der vorliegenden Erfindung ist ein Testsystem zur Erkennung eines erhöhten Vasospasmus-Risikos nach einem Schlaganfall, umfassend:
(a) wenigstens jeweils einen Antikörper gegen GAPDH und Hsc70;
(b) wenigstens einen sekundären Antikörper zum Nachweis von jeweils Antikörper/GAPDH und Antikörper/Hsc70 Komplexen;
(c) wahlweise Mittel zur Quantifizierung der Komplexe;
wobei eine gegenüber einem Referenzwert erhöhte Menge an GAPDH und eine gegenüber einem Referenzwert erniedrigte Menge an Hsc70 ein erhöhtes Vasospasmus-Risiko anzeigt.

Ein weiterer Gegenstand der vorliegenden Erfindung ist ein in vitro Verfahren zum Identifizieren einer pharmazeutisch aktiven Substanz zur Prävention und/oder Behandlung von zerebralem Vasospasmus, umfassend die Schritte:
(a) Bereitstellen einer Zelle, die GAPDH und Hsc70 exprimiert;
(b) In Kontakt bringen einer Testsubstanz mit der Zelle;
(c) Messen der Menge an GAPDH und Hsc70;
wobei eine Reduktion der Menge an GAPDH und eine Erhöhung der Menge an Hsc70 die Testsubstanz als pharmazeutisch aktive Substanz qualifizert.

Besonders bevorzugt kann die pharmazeutisch aktive Substanz ein Arzneimittel oder ein Medizinprodukt sein.

Ein weiterer Gegenstand der vorliegenden Erfindung betrifft ein in vitro Verfahren zum Screenen einer Substanzbibliothek zur Identifizierung einer pharmazeutisch aktiven Substanz zur Prävention und/oder Behandlung von zerebralem Vasospasmus, umfassend die Schritte:
(a) Bereitstellen von Testproben, die Zellen enthalten, die GAPDH und Hsc70 exprimieren;
(b) Messen der Ausgangs-Expressionsmenge an GAPDH und Hsc70;
(c) In Kontakt bringen der Testproben mit Kandidaten der Substanzb ibliothek;
(d) Identifizieren einer Testprobe, die verglichen mit der Ausgangs-Expressionsmenge weniger GAPDH und mehr Hsc70 exprimiert;
(e) Bestimmen des Kandidaten der Substanzbibliothek, der mit der Testprobe in Kontakt gebracht wurde,
wobei der Kandidat der Substanzbibliothek eine pharmazeutisch aktive Substanz ist.

In einer bevorzugten Ausführungsform wird für dieses Verfah ren High-Throughput-Screening (HTS) verwendet.

In einer weiteren bevorzugten Ausführungsform umfasst das Verfahren kolorimetrische, luminometrische, auf Fluoreszenz beruhende oder radioaktive Verfahren.

Eine pharmazeutisch aktive Substanz kann nach einem der beschriebenen Verfahren erhältlich sein.

Es ist ungeklärt, warum ein symptomatischer Vasospasmus bei einigen Patienten auftritt und bei anderen nicht. Zurzeit wird deshalb empfohlen, alle Patienten mit dem Calciumantagonisten Nimodipin zu behandeln. Ursprünglich stand eine angenommene gefäßrelaxierende Wirkung im Vordergrund, heute geht man jedoch von einer direkten neuroprotektiven Wirkung aus. Die Gabe von Nimodipin kann zum einen zu einer Übertherapie von Patienten führen, die keinen Vasospasmus entwickeln werden, zum anderen kann seine blutdrucksenkende Wirkung einen Vasospasmus begünstigen, so dass die bestehende Therapie als nicht ausreichend zu erachten ist. Zur Therapie des symptomatischen Vasospasmus steht derzeit im Grunde nur die sogenannte Tripel-H-Therapie zur Verfügung. Sie besteht aus induzierter Hypertension, Hämodilution und Hypervolämie mittels Katecholaminen und Plasmaexpandern, wird aber wegen der möglichen Nebenwirkungen nicht als Prophylaxe empfohlen. Erst bei Ausbildung von neurologischen Symptomen im Sinne eines Vasospasmus kann somit entsprechend gegengesteuert werden. Insbesondere bei Patienten mit höhergradiger SAB, die klinischneurologisch nur eingeschränkt beurteilt werden können, geht somit wertvolle Zeit verloren. In einem experimentellen Stadium befindet sich die zurzeit in den USA erprobte Frühdilatation der spastischen Gefäße durch einen Ballonkatheter. Durch die vorliegende Erfindung ist es möglich geworden, frühzeitiger als bisher mit einer maximalen Therapie bei ausgewählten Patienten zu beginnen.

Eine pharmazeutisch aktive Substanz umfasst einen Inhibitor von GAPDH, insbesondere einen Inhibitor, der einen Stoffwechselweg beeinflusst, an dem GAPDH beteiligt ist.

Eine pharmazeutisch aktive Substanz umfasst einen Aktivator von Hsc70, insbesondere einen Aktivator, der einen Stoffwechselweg beeinflusst, an dem Hsc70 beteiligt ist.

Ein Kombinationspräparat umfassend einen Inhibitor von GAPDH und einen Aktivator von Hsc70 kann zur Prävention und/oder Prophylaxe von zerebralem Vasospasmus verwendet werden. Das Kombinationspräparat weist den besonderen Vorteil einer erhöhten Wirksamkeit zusammen mit einem reduzierten Risiko für den Patienten in der Prävention und/oder Prophylaxe von zerebralem Vasospasmus auf. Durch das Kombinationspräparat können Einzeldosierungen des Inhibitors von GAPDH sowie des Aktivators von Hsc70 im Vergleich zu den Einzelpräparaten erniedrigt werden. Diese Wirkung ist wahrscheinlich auf einen Kombinationseffekt der beiden Substanzen zurückzuführen.

Im Folgenden sind Beispiele angegeben, die der Erläuterung der Erfindung dienen. Die Beispiele beabsichtigen nicht, den Schutzbereich der Erfindung zu beschränken.

### Verwendetes Material und Methoden

### 1. Humane zerebrale Mikrodialyse

Humanes zerebrales Mikrodialysat wurde nach Genehmigung der Studie durch die zuständige Ethikkommission und Zustimmung der Patienten oder ihrer sorgeberechtigten Verwandten nach dem Fachmann bekannten Standardverfahren gewonnen (Sarrafzadeh et al., Crit Care Med, 30(5) (2002), 1062-1070). Ein flexibler Mikrodialyse-Katheter mit einer formalen Molekulargewichtsschranke von 20 kDa (Membrandurchmesser 0,65 mm, Membranlänge 10 mm) (CMA 70 custom probes, CMA Microdialysis, Solna, Schweden) wurde in das fronto-parietale Parenchym von 97 Patienten implantiert, die alle eine Subarachnoidalblutung (SAB) erlitten hatten. Die Sonden wurden im Anschluss an die operative Aneurysma-Ausschaltung innerhalb 72 Stunden nach dem Blutungszeitpunkt gelegt.

18 der Patienten entwickelten im Verlauf einen symptomatischen Vasospasmus (synonym: verzögertes neurologisches Defizitsyndrom (DINDS)). Die Konzentrationen von Glutamat, Laktat und des Laktat-Pyruvat-Quotienten unterschieden sich signifikant von denen der Patienten, keine Zeichen eines Vasospasmus gezeigt hatten.

Die Mikrodialyse-Katheter wurden mit einer Flussrate von 0,3 µL/min mit einer sterilen Ringerlösung perfundiert und alle 2 Stunden eine Probe entnommen.

### 2. Proteomanalyse

Die Proteinzusammensetzung der Proben wurde mittels der zweidimensionalen Gelelektrophorese (2DE) untersucht. In der ersten Dimension werden alle Proteine der Probe nach ihrem isoelektrischen Punkt, in der zweiten Dimension nach ihrem Molekulargewicht getrennt. Die 2DE wurde nach dem Fachmann bekannten Standardverfahren durchgeführt (Berger et al., Nervenarzt 75 (2004), 113-123; Maurer et al., Proteome Sci 1 (2003), 7). Dazu wurden die Mikrodialyseproben für 5 min in einer Tischzentrifuge bei maximaler Geschwindigkeit zentrifugiert um störende Substanzen zu beseitigen. 5 bis 10 µl (entsprechend etwa 1-2 µg Protein) der Probe wurden in 6 mol/l Harnstoff, 2 M Thioharnstoff, 2 % 3-[(3-Cholamidopropyl)dimethylammonio]-1-propansulfonat (CHAPS), 0,5 % IPG-Puffer pH 3-10 (Amersham Biosciences, Uppsala, Schweden) und wenigen Körnchen Bromphenolblau in einem Gesamtvolumen von 350 µl suspendiert. Die Proben wurden auf Gelstreifen mit einem immobilisierten nichtlinearen pH-Gradienten von pH 3 bis pH 10 (Immobiline DryStrip pH 3-10 NL, 18 cm) aufgetragen und im IPGphor-Apparat (Amersham Biosciences, Uppsala, Schweden) isoelektrisch fokussiert. Das Protokoll für die isoelektrische Fokussierung bestand aus 12 h kussiert. Das Protokoll für die isoelektrische Fokussierung bestand aus 12 h Schwellzeit bei 30 V, dann 200 V, 500 V und 1000 V für je 1 h. Die Spannung wurde dann innerhalb von 30 min auf 8000 V erhöht und bei 8000 V für weitere 12 h konstant gehalten, so dass insgesamt 100300 Vh resultierten.

Die Gelstreifen wurden danach für je 20 min in 1 % Dithiothreitol und 2,5 % Jodazetamid inkubiert und in eine 12,5 % Polyacrylamid-Gelelektrophorese in Gegenwart von 0,1 % Natriumdodecylsulfat überführt. Die 20 x 20 cm² großen Gele wurden bei 30 mA für 30 min und 100 mA für etwa 4 h in einer geeigneten Gelkammer elektrophoriert. Die Gele wurden mit einer hochsensitiven Silberfärbung zum Sichtbarmachen der Proteinspot entwickelt (Blum et al., Electrophoresis 8 (1987), 93-99), digitalisiert und densitometrisch mit der Phoretix 2D Elite Software, Version 6.01 (Nonlinear Dynamics, Newcastle-upon-Tyne, UK) ausgewertet. Einzelne Proteinspots wurden in den Gelen detektiert und ihr Spotvolumen, definiert als Integral der Optischen Dichte über der Spotfläche, bestimmt.

Für jeden Patienten wurden 2 Gelproben, die zu unterschiedlichen Zeitpunkten entnommen worden waren, untersucht.

### 3. Hierarchische Cluster-Analyse

Die Rohdaten der Spotvolumina wurden durch das im Internet verfügbare Unterprogramm EPCLUST Version 0.9.23 beta des EMBL-EBI Programms "Expression Profiler", das unter der URL http://ep.ebi.ac.uk aufgerufen werden kann (Vilo et al., The analysis of gene expression data: Methods and software (2003)) untersucht. Die Daten wurden log2-transformiert und für jeden Spot mittelwertzentriert. Alle Daten wurden aus den 20 Gelen (je 2 Proben von 10 Patienten) in die Analyse eingeschlossen. Die Parameter des hierarchischen Clusterns wurden auf "average linkage (average distance, UPGMA)" festgesetzt, und lineare Korrelation basierend auf der linearen Korrelationsdistanz zentriert nach Pearson wurde verwendet. Die Dendrogramm-Schnitthöhe wurde mit 0,5 gewählt.

### Ergebnisse

Es wurden durchschnittlich 57 ± 22 Proteinspots (N=20) in den individuellen Gelen mit einem Minimum von 37 bis zu einem Maximum von 149 Proteinspots gefunden. Die hierarchische Clusteranalyse der Daten zeigte 2 Gruppen differenziell exprimierter Proteine, die durch Vergleich mit der Proteomreferenzkarte für humanes Mikrodialysat (Maurer et al., Proteome Sci 1 (2003), 7) als Glyzeraldehyd-3-phosphat-Dehydrogenase (GAPDH) und das Protein Heat-shock-cognate-70 kDa (Hsc70) identifiziert wurden.

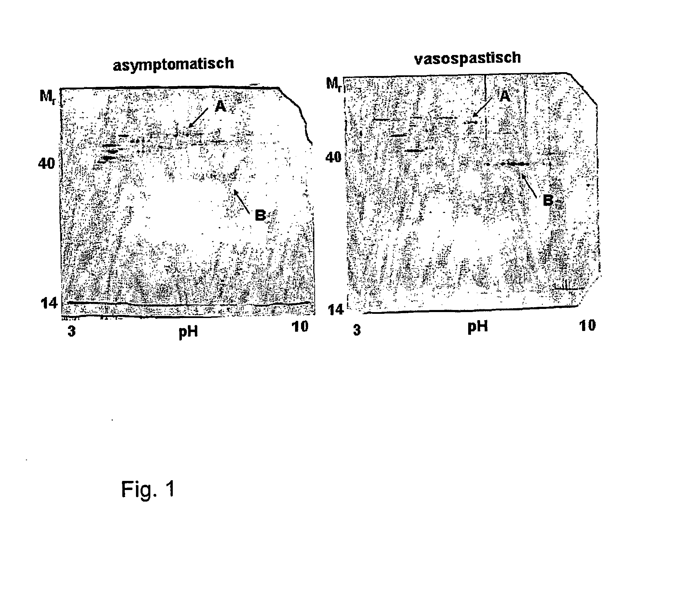

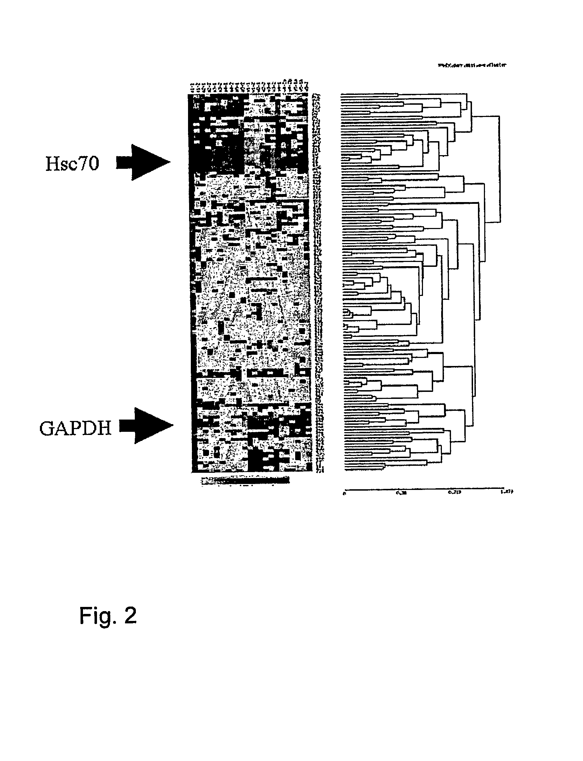

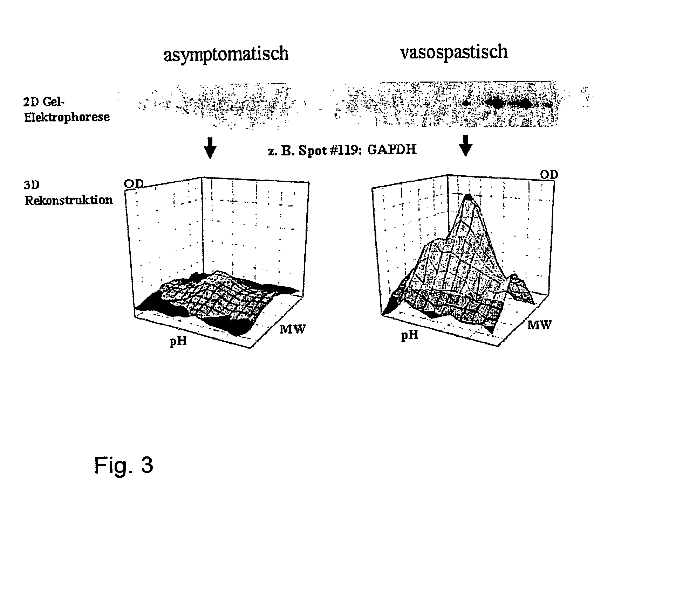

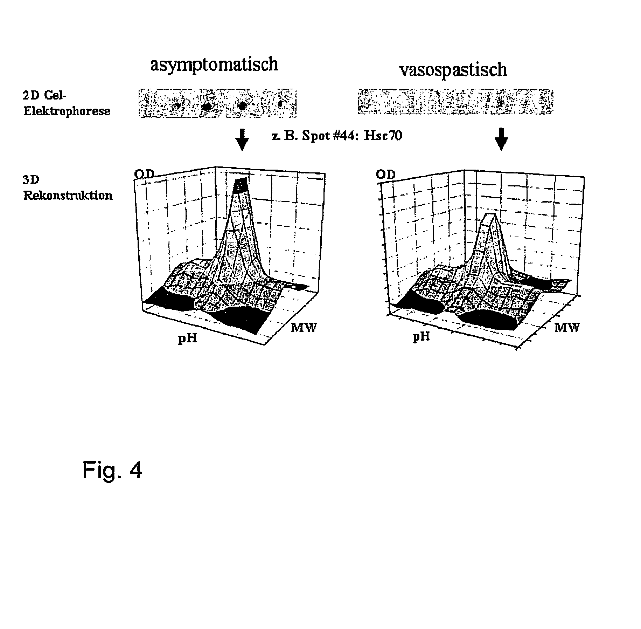

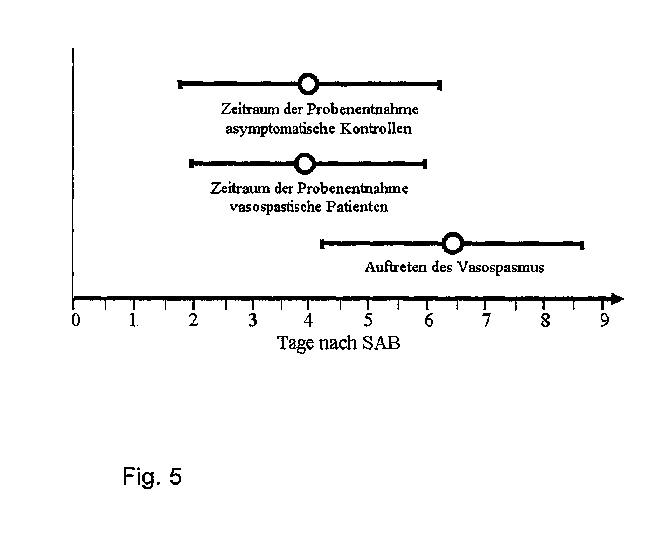

Die einzelnen Ergebnisse sind in den beigefügten Figuren 1 bis 5 gezeigt und in der Kurzbeschreibung der Figuren detailliert beschrieben.

### Referenzliste

1. Benveniste, J Neurochem 52 (1989), 1667-1679
2. Berger et al., Nervenarzt 75 (2004), 113-123
3. Blum et al., Electrophoresis 8 (1987), 93-99
4. Bulliard et al., Biochem J 324 (Pt 2) (1997), 555-563
5. Chuang und Ishitani, Nat Med 2 (1996), 609-610
6. Dastoor und Dreyer, J Cell Sci 113 (Pt 16) (2000), 2845-2854
7. Dietrich und Dacey, Neurosurgery 46 (2000), 517-530
8. Dumont et al., J Neurosurgery 96 (2002), 985-986
9. Dumont et al., Neurosurgery 53 (2003), 123-133
10. Görg et al., Electrophoresis 21 (2000), 1037-1053
11. Graven et al., J Biol Chem 269 (1994), 24446-24453
12. Graven et al., Am J Physiol 274 (1998), C347-C355
13. Hijdra et al., Stroke 18 (1987), 1061-1067
14. Janjua und Mayer, Curr Opin Crit Care 9 (2003), 113-119
15. Kragten et al., J Biol Chem 273 (1998), 5821-5828
16. Laher und Zhang, J Cereb Blood Flow Metab 21 (2001), 887-906
17. Maurer et al., Proteome Sci 1 (2003), 7
18. Myung et al., Mol Genet Metab 80 (2003), 444-450
19. Nakamura et al., Biochem Biophys Res Commun 290 (2002), 858-864
20. Peerdeman et al., J Neurol 250 (2003), 797-805
21. Persson und Hillered, J Neurosurg 76 (1992), 72-80
22. Rabilloud, Proteomics 2 (2002), 3-10
23. Sakowitz et al., J Cereb Blood Flow Metab 21 (2001), 1067-1076
24. Sarrafzadeh et al., Crit Care Med 30 (2002), 1062-1070
25. Sarrafzadeh et al., Curr Neurol Neurosci Rep 3 (2003), 517-523
26. Sarrafzadeh et al., Stroke 35 (2004), 638-643
27. Saveland et al., Neurosurgery 38 (1996), 12-19
28. Sawa et al., Proc Natl Acad Sci USA 94 (1997),11669-11674
29. Sirover, J Cell Biochem 66 (1997), 133-140
30. Sirover, Biochim Biophys Acta 1432 (1999), 159-184
31. Sobey, Clin Exp Pharmacol Physiol 28 (2001), 926-929
32. Sunaga et al., Neurosci Lett 200 (1995), 133-136
33. Tatton et al., J Neural Transm Suppl (2000), 77-100
34. Tatton et al., J Neural Transm 110 (2003), 509-515
35. Ungerstedt, Measurement of neurotransmitter release in vivo. 6, Marsden CA Hrsg. Chishester, Wiley (1984), 81-105
36. Ungerstedt, J Int Med 230 (1991), 365-373
37. Unterberg et al., J Neurosurgery 94 (2001), 740-749
38. Vilo J et al., Expression profiler. In: The analysis of gene expression data: Methods and software (2003), Parmigiani G et al., Hrsg. New York, NY, Springer Verlag

## Patentansprüche

1. In vitro Verfahren zur Diagnose eines erhöhten Vasospasmus-Risikos eines Patienten, umfassend die Schritte:
(a) Bereitstellen einer Probe des Patienten;
(b) Bestimmen der Menge an Glyzeraldehyd-3-phosphat-Dehydrogenase Protein (GAPDH) und Heat-shock-cognate 70 kDa Protein (Hsc70) in der Testprobe;
(c) Vergleichen der Menge an GAPDH und Hsc70 mit einem Referenzwert;
wobei eine gegenüber dem Referenzwert erhöhte GAPDH Menge und eine gegenüber dem Referenzwert verringerte Hsc70 Menge ein erhöhtes Vasospasmus-Risiko anzeigt.

2. Verfahren nach Anspruch 1, wobei die Probe ein zerebrales Mikrodialysat, Liquor cerebrospinalis, Blutplasma oder Blutserum ist.

3. Verfahren nach Anspruch 1 oder 2, wobei das erhöhte Vasospasmus-Risiko nach einer Subarachnoidalblutung (SAB) oder nach einer Gehirnmassenblutung auftritt.

4. Verfahren nach einem der vorhergehenden Ansprüche, wobei die Bestimmung der Menge an GAPDH und Hsc70 durch eine Proteinkonzentrationsbestimmung erfolgt.

5. Verfahren nach Anspruch 4, wobei die Proteinkonzentrationsbestimmung durch einen Antikörper oder durch zweidimensionale Gelelektrophorese erfolgt.

6. Verfahren nach einem der vorhergehenden Ansprüche, wobei der Vasospasmus zerebraler Vasospasmus ist.

7. In vitro Verwendung von GAPDH und Hsc70 zur Diagnose eines erhöhten Vasospasmus-Risikos.

8. Verwendung nach Anspruch 7, wobei der Vasospasmus zerebraler Vasospasmus ist.

9. Testsystem zur Erkennung eines erhöhten Vasospasmus-Risikos nach einem Schlaganfall, umfassend:
(a) wenigstens jeweils einen Antikörper gegen GAPDH und Hsc70;
(b) wenigstens einen sekundären Antikörper zum Nachweis von jeweils Antikörper/GAPDH und Antikörper/Hsc70 Komplexen;
(c) Mittel zur Quantifizierung der Komplexe;
wobei eine gegenüber einem Referenzwert erhöhte Menge an GAPDH und eine gegenüber einem Referenzwert erniedrigte Menge an Hsc70 ein erhöhtes Vasospasmus-Risiko anzeigt.

10. In vitro Verfahren zum Identifizieren einer pharmazeutisch aktiven Substanz zur Prävention und/oder Behandlung von zerebralem Vasospasmus, umfassend die Schritte:
(a) Bereitstellen einer Zelle, die GAPDH und Hsc70 exprimiert;
(b) In Kontakt bringen einer Testsubstanz mit der Zelle;
(c) Messen der Menge an GAPDH und Hsc70;
wobei eine Reduktion der Menge an GAPDH und eine Erhöhung der Menge an Hsc70 die Testsubstanz als pharmazeutisch aktive Substanz qualifiziert.

11. Verfahren nach Anspruch 10, wobei die pharmazeutische aktive Substanz ein Arzneimittel ist.

12. Verfahren nach Anspruch 10 zum Screenen einer Substanzbibliothek zur Identifizierung der pharmazeutisch aktiven Substanz zur Prävention und/oder Behandlung von zerebralem Vasospasmus, wobei die Zelle in Testproben enthalten ist und die Testsubstanz ein Kandidat der Substanzbibliothek ist.

13. Verfahren nach Anspruch 12, wobei das Verfahren High-Throughput-Screening (HTS) umfasst.

14. Verfahren nach Anspruch 12 oder 13, wobei das Verfahren kolorimetrische luminometrische, auf Fluoreszenz beruhende oder radioaktive Verfahren umfasst.

## Claims

1. In vitro method for the diagnosis of an increased risk of vasospasm in a patient, comprising the steps:
(a) providing a sample from the patient;
(b) determining the amount of glyceraldehyde-3-phosphate dehydrogenase protein (GAPDH) and heat shock cognate 70 kDa protein (Hsc70) in the test sample;
(c) comparing the amount of GAPDH and Hsc70 with a reference value;
wherein an increased amount of GAPDH relative to the reference value and a reduced amount of Hsc70 relative to the reference value indicates an increased risk of vasospasm.

2. Method according to claim 1, wherein the sample is a cerebral microdialysate, Liquor cerebrospinalis, blood plasma or blood serum.

3. Method according to claim 1 or 2, wherein the increased risk of vasospasm occurs after a subarachnoid haemorrhage (SAH) or after a massive brain haemorrhage.

4. Method according to any one of the preceding claims,
wherein the determination of the amount of GAPDH and Hsc70 is effected by determining the protein concentration.

5. Method according to claim 4, wherein the determination of the protein concentration is effected by an antibody or by two-dimensional gel electrophoresis.

6. Method according to any one of the preceding claims,
wherein the vasospasm is cerebral vasospasm.

7. In vitro use of GAPDH and Hsc70 for the diagnosis of an increased risk of vasospasm.

8. Use according to claim 7, wherein the vasospasm is cerebral vasospasm.

9. Test system for recognising an increased risk of vasospasm after a stroke, comprising:
(a) at least one antibody against GAPDH and at least one antibody against Hsc70;
(b) at least one secondary antibody for detecting antibody/GAPDH and antibody/Hsc70 complexes, respectively;
(c) means for quantifying the complexes;
wherein an increased amount of GAPDH relative to a reference value and a reduced amount of Hsc70 relative to a reference value indicates an increased risk of vasospasm.

10. In vitro method for identifying a pharmaceutically active substance for the prevention and/or treatment of cerebral vasospasm, comprising the steps:
(a) providing a cell which expresses GAPDH and Hsc70;
(b) bringing a test substance into contact with the cell;
(c) measuring the amount of GAPDH and Hsc70;
wherein a reduction in the amount of GAPDH and an increase in the amount of Hsc70 qualifies the test substance as a pharmaceutically active substance.

11. Method according to claim 10, wherein the pharmaceutically active substance is a medicament.

12. Method according to claim 10 for screening a substance library in order to identify the pharmaceutically active substance for the prevention and/or treatment of cerebral vasospasm, wherein the cell is contained in test samples and the test substance is a candidate from the substance library.

13. Method according to claim 12, wherein the method comprises high throughput screening (HTS).

14. Method according to claim 12 or 13, wherein the method comprises colorimetric, luminometric, fluorescence-based or radioactive methods.

## Revendications

1. Procédé pour le diagnostic in vitro d'un risque accru de vasospasme chez un patient, comportant les étapes suivantes :
(a) mise à disposition d'un échantillon du patient ;
(b) détermination de la quantité de la protéine Glycéraldéhyde-3-Phosphate Déshydrogénase (GAPDH) et de la protéine Heat-Shock-cognate 70 kDa (Hsc70) dans l'échantillon d'essai ;
(c) comparaison de la quantité de GAPDH et de HSC70 avec une valeur de référence ;
dans lequel une quantité de GAPDH augmentée par rapport à la valeur de référence et une quantité de Hac70 réduite par rapport à la valeur de référence indiquent un risque de vasospasme.

2. Procédé selon la revendication 1, dans lequel l'échantillon est un microdyalisat cérébrale, du liquide cérébro-spinal, du plasma sanguin ou du sérum sanguin.

3. Procédé selon la revendication 1 ou 2, dans lequel le risque accru de vasospasme se manifeste suite à une hémorragie sous-arachnoïdale (SAB) ou suite à une hémorragie de la masse cérébrale.

4. Procédé selon l'une des revendications précédentes, dans lequel que la détermination de la quantité de GAPDH et de Hsc70 est effectuée au moyen d'une détermination de la concentration protéique.

5. Procédé selon la revendication 4, dans lequel la détermination de la concentration protéique s'effectue soit au moyen d'un anticorps soit au moyen d'une électrophorèse bidimensionnelle sur gel.

6. Procédé selon l'une des revendications précédentes, dans lequel le vasospasme est un vasospasme cérébral.

7. Utilisation in vitro de GAPDH et de Hsc70 pour le diagnostic d'un risque accru de vasospasme.

8. Utilisation selon la revendication 7, dans laquelle le vasospasme est un vasospasme cérébral.

9. Système d'analyse pour l'identification d'un risque accru de vasospasme suite à une apoplexie, comportant :
(a) au moins un anticorps contre GAPDH et Hsc70 respectivement ;
(b) au moins un anticorps secondaire pour la détection de complexes d'anticorps GAPDG et d'anticorps Hsc70 respectivement ;
(c) un moyen de quantification des complexes ;
dans lequel une quantité de GAPDH augmentée par rapport à une valeur de référence et une quantité de Hac70 réduite par rapport à une valeur de référence indiquent un risque accru de vasospasme.

10. Procédé in vitro pour l'identification d'une substance pharmaceutiquement active pour la prévention et/ou le traitement du vasospasme cérébral, comportant les étapes de :
(a) mettre à disposition une cellule qui exprime la GAPDH et la Hsc70 ;
(b) mettre en contact une substance d'essai avec la cellule ;
(c) mesurer la quantité de GAPDH et de Hsc70 ;
dans lequel une réduction de la quantité de GAPDH et une augmentation de la quantité de Hsc70 qualifie la substance d'essai de substance pharmaceutiquement active.

11. Procédé selon la revendication 10, dans lequel la substance pharmaceutiquement active est un médicament.

12. Procédé selon la revendication 10 pour le criblage d'une bibliothèque de substances visant à identifier la substance pharmaceutiquement active en vue de la prévention et/ou du traitement du vasospasme cérébral, dans lequel la cellule est contenue dans les échantillons d'essai d'analyse et la substance d'essai est une candidate de la bibliothèque de substances.

13. Procédé selon la revendication 12, dans lequel le procédé comporte le criblage à haut débit (HTS).

14. Procédé selon la revendication 12 ou 13 dans lequel le procédé comporte un procédé colorimétrique, luminométrique, par fluorescence ou radioactif.
